# EUROPÄISCHE PATENTSCHRIFT

(11) **EP 0 698 017 B1**
(45) Veröffentlichungstag und Bekanntmachung des Hinweises auf die Patenterteilung: **11.12.1996**
(21) Anmeldenummer: 94916183.0
(22) Anmeldetag: 02.05.1994
(51) Int. Cl.: C07D 285/16, A01N 43/88

(54) **MAGNESIUMSALZ VON 3-ISOPROPYL-2,1,3-BENZOTHIADIAZIN-4-ON-2,2-DIOXID, VERFAHREN ZU SEINER HERSTELLUNG UND SEINE VERWENDUNG ZUR BEKÄMPFUNG VON UNERWÜNSCHTEM PFLANZENWUCHS**
MAGNESIUM SALT OF 3-ISOPROPYL-2,1,3-BENZOTHIADIAZIN-4-ONE-2,2-DIOXIDE, METHOD OF PREPARING IT AND ITS USE IN THE CONTROL OF UNDESIRABLE PLANT GROWTH
SEL DE MAGNESIUM DU 3-ISOPROPYL-2,1,3-BENZOTHIADIAZIN-4-ONE-2,2-DIOXYDE, PROCEDE DE PREPARATION ET SON UTILISATION POUR EVITER LA CROISSANCE DES VEGETAUX INDESIRABLES

(30) Priorität: 12.05.1993 DE 4315878
(43) Veröffentlichungstag der Anmeldung: 28.02.1996
(73) Patentinhaber: BASF Aktiengesellschaft, 67063 Ludwigshafen (DE)
(72) Erfinder: JAEGER, Karl-Friedrich, D-67117 Limburgerhof (DE); PARG, Adolf, D-67098 Bad Dürkheim (DE); DUREIN, Alfons, D-67354 Römerberg (DE)
(86) Internationale Anmeldenummer: EP9401391
(87) Internationale Veröffentlichungsnummer: WO9426727

(56) Entgegenhaltungen:
- EP-A- 0 207 653
- DE-A- 1 542 836
- DE-A- 2 164 459
- DE-A- 2 217 722
- CHEMICAL ABSTRACTS, vol. 115, no. 9, 2. September 1991, Columbus, Ohio, US; abstract no. 92230u, V.F. SHULGIN ET AL. 'Synthesis and study of 3d-metal salts with 3-isopropylbenzo-2,1,3-thiadiazin-4-one 2,2-dioxide' Seite 762 ;

## Beschreibung

Die vorliegende Erfindung betrifft ein festes, nicht-hygroskopisches Magnesiumsalz von 3-Isopropyl- 2,1,3-benzothiadiazin-4-on-2,2-dioxid I.

Außerdem betrifft die Erfindung Feststoff-Formulierungen dieses Salzes, Verfahren zur Herstellung dieses Salzes, Mittel, insbesondere Pulver und Granulate sowie wasserlösliche oder wasserdispergierbare Folienbeutel, die dieses Salz enthalten und deren Verwendung zur Bekämpfung von unerwünschtem Pflanzenwachstum.

Aus der Literatur sind Benzothiadiazin-4-on-2,2-dioxide und deren Salze als Herbizide bekannt (DE-A 15 42 836, DE-A 21 64 459, DE-A 22 17 722). Im Hinblick auf die Verwendung von Salzen werden in der DE-A 15 42 836, der DE-A 21 64 459 und der DE-A 22 17 722 in allgemeiner Form auch Erdalkalimetallsalze als Anwendungsform genannt, wobei die Kalziumsalze besondere Erwähnung finden. Besondere Eigenschaften insbesondere der Magnesiumsalze sind diesen Schriften jedoch nicht zu entnehmen.

Die Verwendung von wasserlöslichen oder wasserdispergierbaren Folienbeuteln zur für den Anwender sicheren Handhabung von Pflanzenschutzmitteln ist beispielsweise aus der EP-A 449 773 und der EP-A 493 553 bekannt.

3-Isopropyl-2,1,3-benzothiadiazin-4-on-2,2-dioxid (INN-Name: Bentazon) wird üblicherweise als hochkonzentrierte wäßrige Lösung formuliert, wobei das Natriumsalz oder das Diethanolammoniumsalz wegen seiner günstigeren Lösungseigenschaften gegenüber der Säure bevorzugt Verwendung findet. Bei der Anwendung werden diese Bentazonlösungen mit Wasser verdünnt und mittels Spritzapplikation ausgebracht.

In Hinblick auf eine einfache und sichere Anwendung von Bentazon-Formulierungen und im Hinblick auf eine einfache Entsorgung der Verpackungen ist eine Feststoff-Formulierung (beispielsweise Granulat-Formulierung) wünschenswert.

Staubfreie Granulatformulierungen können einfach und sicher angewendet werden. Gegenüber den bisher verwendeten Flüssigformulierungen ergeben sich Vorteile durch einen verminderten Packmitteleinsatz und sichere Lagerung. Bei tiefen Lagertemperaturen kann es bei Flüssigprodukten zu unerwünschtem Auskristallisieren kommen.

Außerdem können Feststoff-Formulierungen in wasserlöslichen oder wasserdispergierbaren Folienbeuteln abgepackt werden. Bei der Anwendung wird der Folienbeutel mit Produkt im Spritztank aufgelöst oder dispergiert. Eine mit Produktresten kontaminierte Verpackung wird dadurch vermieden.

Natrium-, Kalzium- und Kaliumbentazon haben den Nachteil sehr hygroskopisch zu sein. Dies führt im Fall von Feststoff-Formulierungen dazu, daß das Produkt bereits unter Einfluß der Luftfeuchtigkeit mit der Zeit verklumpt oder sogar zerfließt und dadurch nicht mehr ohne weiteres zu dosieren ist. Selbst die Einbringung dieser Verbindungen in wasserlösliche Folienbeutel bietet keinen Vorteil, da durch Wechselwirkung der hygroskopischen Wirkstoffe einerseits und der Folien andererseits die Folien dehydratisiert werden. Dies hat zur Folge, daß die Folien verspröden, d.h. die Lagerstabilität ist nicht mehr gewährleistet. Das Diethanolaminsalz von Bentazon kann nicht zu einem Festprodukt getrocknet werden (Schmelzpunkt < 20°C).

Aufgabe der vorliegenden Erfindung war daher die Bereitstellung einer lagerstabilen, nicht-hygroskopischen Feststoff-Formulierung des Wirkstoffs Bentazon.

Demgemäß wurden das eingangs genannte Salz, Feststoff-Formulierungen dieses Salzes, Verfahren zur seiner Herstellung, Mittel, insbesondere Pulver und Granulate sowie wasserlösliche Folienbeutel, die dieses Salz enthalten und dessen Verwendung zur Bekämpfung von unerwünschtem Pflanzenwachstum gefunden.

Unter dem Begriff "nicht-hygroskopisch" ist im Sinne der vorliegenden Erfindung zu verstehen, daß das Salz I bei einer Temperatur von 20°C und einer relativen Luftfeuchte von 50 % im Gleichgewicht mit der feuchten Luft nicht verklumpt, sondern fließfähig und damit dosierbar bleibt. Ebenso dehydratisiert das Salz I nicht die wasserlöslichen Folien, d.h. die Folienbeutel bleiben auch bei längerer Lagerung elastisch und damit stabil.

Die Herstellung von 3-Isopropyl-2,1,3-benzothiadiazin-4-on-2,2-dioxid ist aus der eingangs zitierten Literatur bekannt.

Man erhält das Magnesiumsalz dadurch, daß man 3-Isopropyl-2,1,3-benzothiadiazin-4-on-2,2-dioxid (II) in wäßriger Lösung mit einer Magnesiumverbindung behandelt oder extrahiert, anschließend den Feststoff in an sich bekannter Weise isoliert und trocknet.

Die Umsetzungstemperatur ist für die Salzbildung nicht kritisch und hat lediglich einen Einfluß auf die Löslichkeit der verwendeten Ausgangsstoffe und damit die Umsetzungskonzentration. Demgemäß kann die Salzbildung bei Temperaturen von 10°C bis zum Siedepunkt der Lösung erfolgen.

Als Magnesiumverbindungen finden üblicherweise Hydroxyde, Oxide, Carbonate und Hydrogencarbonate Verwendung.

Üblicherweise werden diese Magnesiumverbindungen in äquimolaren Mengen bezogen auf (II) verwendet. Es kann zur Vervollständigung der Umsetzung vorteilhaft sein, die anorganischen Verbindungen in einem Überschuß zu verwenden. Dieser Überschuß sollte wiederum im Hinblick auf die unerwünschte Belastung der zurückerhaltenen Abwässer mit Salzen gering gehalten werden. Üblicherweise braucht der Überschuß somit 10 mol-% nicht zu übersteigen.

Dabei kann II direkt in wäßriger Lösung mit den vorstehend genannten Salzen umgesetzt werden oder II wird aus einer organischen Lösung in die Wasserphase, welche die Magnesiumsalze enthält, extrahiert.

Daneben ist es auch möglich, eine wäßrige oder organische Lösung eines Ammonium- oder Alkalimetallsalzes der Verbindung II mit einem Magnesiumsalz umzusetzen.

Die Isolierung des festen Salzes erfolgt in an sich bekannter Weise durch Kristallisation und/oder durch Trocknung der wäßrigen Lösung von I nach allgemein üblichen Verfahren. Als Beispiele seien die Wirbelbeschichttrocknung, Sprühtrocknung oder Vakuumtrocknung genannt.

Zur Herstellung eines Granulats geschieht die Trocknung vorteilhafterweise nach dem Wirbelbett-Verfahren oder durch Agglomeration eines Pulvers von I, das durch Sprühtrocknung oder Vakuumtrocknung hergestellt wird.

Die so erhaltenen Granulate bestehen üblicherweise aus 20 bis 100 % an Magnesiumsalz I. Die Korngröße dieser Granulate liegt im allgemeinen bei 200 µm bis 3000 µm. Der Staubanteil der Granulate ist gering. Der Staubgehalt einer 30 g Probe ist kleiner als 20 mg (CIPAC MT 171: "Dustiness of Granular Formulations") wodurch eine hohe Sicherheit für den Anwender erreicht wird. Das Schüttgewicht derartiger Granulate liegt bei 400 - 800 g/l.

Es ist außerdem möglich das Magnesiumsalz von 3-Isopropyl-2,1,3-benzothiadiazin-4-on-2,2-dioxid, sei es kristallin, in Form von Pulver oder als Granulat, in wasserlösliche oder wasserdispergierbare Folienbeutel einzubringen und auf diesem Wege eine sichere Handhabung durch die Anwender zu erreichen und kontaminierte Verpackungen nach der Anwendung zu vermeiden.

Die gefüllten Folienbeutel enthalten üblicherweise 0,1 bis 10 kg, vorzugsweise 0.5 bis 5 kg an Wirkstoff (I). Sie werden üblicherweise vollständig mit dem Wirkstoff, einer Wirkstoffaufbereitung oder einer Wirkstoffmischung gefüllt. Es ist aber auch möglich, daß die Folienbeutel ein größeres Volumen aufweisen, so daß zusatzstoffe und/oder andere Wirkstoffe zugegeben werden können. Die Folienbeutel sind bei üblichen Temperaturen und Luftfeuchtigkeiten fest und flexibel und an mindestens einer Seite verschweißt. Die Stärke der Folien beträgt 20 bis 100 micron, vorzugsweise 30 bis 60 micron. Der Wassergehalt in den polymeren Folien kann bis zu 20 % betragen.

Derartige Folienbeutel an sich sind z.B. bekannt aus EP-A 449 773 oder EP-A 493 553. Die zu ihrer Herstellung verwendeten Polymere sind beispielsweise
- polymere Polyvinylalkohole, vorzugsweise Polyvinylalkoholpolymere mit polyvalenten Alkoholen,
- polymere Polyvinylalkohole, vorzugsweise Polyvinylakoholpolymere mit polyvalenten Alkoholen,
- Methylcellulose,
- Ethylenoxyd Copolymere,
- Polymere von Vinylpyrrolidon oder Vinylacetat,
- Gelatine, Carboxylmethylcellulose, Dextrose, Hydroxyethylcellulose oder
- mit polyvalenten Alkoholen wie Ethylenglycol, Propylenglycol, Glycerol, Sorbitol und anderen kombinierte Methylcellulose.

Zum Schutz der Folienbeutel kann es vorteilhaft sein, diese in größere Container oder Pakete zu verpacken. Als Verpackungsmaterial eignen sich billige Materialien wie Plastik, Papier, Karton oder Aluminium. Derartige Verpackungen sind aus Sicht der Umweltbelastung und der Entsorgung unbedenklich, da sie nicht mit den Pflanzenschutzwirkstoffen in Kontakt kommen. Außerdem besteht die Möglichkeit, derartige Verpackungen wiederzuverwenden, wodurch der Rohstoffbedarf und damit die Belastung der Umwelt weiter reduziert werden kann.

Die wie vorstehend erhaltenen Granulate oder die gefüllten Folienbeutel können außer den Salzen I noch weitere übliche Zusatzstoffe, z.B. oberflächenaktive Stoffe, Füllstoffe, oder auch weitere Pflanzenschutzwirkstoffe enthalten.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanole oder Fettalkoholglycolethern, Kondensationsprodukte von sulfoniertem Naphthalin und seinen Derivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol- oder Tributylphenylpolyglycolether, Alkylarylpolyetheralkohole, Isotridecylalkohol, Fettalkoholethylenoxid- Kondensate, ethoxyliertes Rizinusöl, Polyoxyethylenalkylether oder Polyoxypropylen, Laurylalkoholpolyglycoletheracetat, Sorbitester, Lignin-Sulfitablaugen oder Methylcellulose in Betracht.

Als Füllstoffe bzw. feste Trägerstoffe dienen beispielsweise Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Kalzium- und Magnesiumsulfat, Magnesiumoxid, gemahlene Kunststoffe, sowie Düngemittel wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Formulierungen enthalten im allgemeinen 20 bis 100 Gew.-%, vorzugsweise 50 bis 100 Gew.-% des Salzes I. Das Magnesiumsalz I findet dabei in einer Reinheit von 90 % bis 100 %, vorzugsweise 95 % bis 100 % (nach NMR/HPLC/GC-Spektrum), Verwendung.

Die wie vorstehend beschriebenen Granulate oder die mit den Salzen I gefüllten wasserlöslichen oder in Wasser dispergierbaren Folienbeutel können vom Anwender zum Bereiten wäßriger Lösungen verwendet werden, welche dann in der für Bentazon oder für die entsprechende Mischung üblichen Weise zur Bekämpfung von unerwünschten Pflanzen eingesetzt werden.

Die Applikation der so erhaltenen wäßrigen Lösungen kann in üblicher Weise im Vorauflauf- oder im Nachauflaufverfahren erfolgen. Sind die Wirkstoffe für gewisse Kulturpflanzen weniger verträglich, so können bei der Nachauflaufanwendung Ausbringungstechniken angewandt werden, bei welchen die herbiziden Mittel mit Hilfe der Spritzgeräte so appliziert werden, daß die Blätter der empfindlichen Kulturpflanzen nach Möglichkeit nicht benetzt werden, während die Wirkstoffe auf die Blätter von darunter wachsenden unerwünschten Pflanzen oder auf die unbedeckte Bodenfläche gelangen (post-directed, lay-by).

Die Aufwandmengen an Wirkstoff betragen je nach Bekämpfungsziel, Jahreszeit, Zielpflanze und Wachstumsstadium 0,001 bis 5,0 kg/ha, vorzugsweise 0,01 bis 1,0 kg/ha an Salz I.

### Chemische Beispiele:

1. Herstellung von Magnesiumbentazon
1.a. In ein Gemisch, bestehend aus 24 Teilen Bentazon in 216 Teilen 1,2-Dichloräthan und 300 Teilen Wasser werden unter Rühren 2,9 Teile Magnesiumhydroxid eingetragen und bei 60°C gerührt. Nach ca. 5 h erhält man eine klare, wäßrige Phase. Nach Phasentrennung wird die wäßrige Phase bei 50-60°C unter Vakuum zur Trockene eingedampft. Man erhält 30,1 Teile Magnesiumbentazon.
1.b.Versuch entsprechend Beispiel 1.a. wobei das Magnesiumhydroxid durch 2,02 Teile Magnesiumoxid ersetzt wird.
1.c. In eine Suspension aus 24 Teilen Bentazon (II) und 300 Teilen Wasser werden unter Rühren 2,9 Teile Magnesiumhydroxid eingetragen und bei 50°C für 2 Stunden gerührt. Die erhaltene wäßrige Lösung wird bei 50-60°C unter Vakuum zur Trockene eingedampft. Man erhält 30 Teile Magnesiumbentazon.

2. Beispiele für Feststoff-Formulierung
2.a. Herstellung eines Granulats mit Magnesiumbentazon
   Eine 40%ige wäßrige Magnesiumbentazonlösung wird auf einem Wirbelschichtsprühgranulator bei 120°C Trocknungsluft-Temperatur getrocknet. Dabei wird die Magnesiumbentazonlösung in die Wirbelschicht eingedüst und es entstehen Granulatpartikel durch Agglomeration und Trocknung. Das Granulat besteht aus 82 % Magnesiumbentazon und hat einen Wassergehalt von 18 %. Die mittlere Granulatkorngröße beträgt 0,5 mm. Das Granulat ist staubfrei und löst sich schnell in Wasser auf. Es ist nicht hygroskopisch und bleibt an feuchter Luft fließfähig und dosierbar.
2.b.Herstellung eines Pulvers mit 75 % Magnesiumbentazon
   In einer Lösung aus 30 Teilen Magnesiumbentazon in 70 Teilen Wasser werden 6 Teile Natriumligminsulfonat unter Rühren gelöst. Diese Lösung wird auf einem Sprühturm bei 160°C Trocknungslufttemperatur sprühgetrocknet. Man erhält ein Pulver mit 75 % Magnesiumbentazon.

3. Herstellung von wasserlöslichen Folienbeuteln
3.a. 430 g Magnesiumbentazon werden in wasserlösliche KB-Folie (Fa. Aicello Chem. Co., Ltd., Japan) abgepackt und die Folie dicht verschweißt.
   In ein Spritzgerät werden 75 1 Wasser mit einer Temperatur von 10°C eingefüllt und mittels einer Pumpe umgewälzt.
   Die gefüllten Folienbeutel werden in das Spritzgerät eingebracht. Bei einer Wassertemperatur von 14°C sind Produkt und Folie nach 2 Minuten vollständig aufgelöst.

4. Physikalisches Verhalten
4.a.Untersuchung zur Hygroskopizität der Salze
Jeweils 1 g an Substanz wurden für 48 Stunden bei 50°C im Vakuum getrocknet. Die getrockneten Proben wurden bei 55 % und 65 % relativer Luftfeuchte und 20°C Temperatur aufbewahrt und die Gewichtszunahme der Proben nach Erreichen des Gleichgewichtszustandes gemessen. Ebenso wurden die Fließeigenschaften der Proben und deren Aussehen beurteilt. Hinsichtlich der Hygroskopizität kritische Substanzen nahmen viel Wasser aus der Luft bis zum Erreichen des Gleichgewichtszustandes auf. Dies führte zum Verbacken der Substanzen. Die Ergebnisse sind in der folgenden Tabelle zusammengestellt.

| Art des Salzes | rel. Luftfeuchte | Gewichtszunahme in Gew.-% | Eigenschaften nach der Lagerung |
|---|---|---|---|
| Natriumsalz | 55 % | 12,6 % | verklumpt, verbacken |
| Kaliumsalz | 55 % | 6,7 % | verklumpt, verbacken |
| Kalziumsalz | 55 % | 12,0 % | verklumpt, verbacken |
| Magnesiumsalz | 55 % | 2,6 % | kristallin, fließfähig |
| | 65 % | 2,9 % | kristallin, fließfähig |

4.b) Untersuchung zum Verhalten der Salze im Folienbeutel:
Jeweils 10 g an Substanz in Form eines Granulats in Folienbeutel eingeschweißt. Die gefüllten Folienbeutel (Folie: Monosol 8030, Hersteller: Chris Craft Inc., USA) wurden dann 4 Wochen bei verschiedenen Temperaturen in wasserdampfdichter Umverpackung aufbewahrt. Die Stabilität der Folien drückt sich durch die Elastizität der Folien bei mechanischer Beanspruchung aus. Wenn durch das Bentazonsalz Wasser aufgenommen wird, gibt die Folie entsprechende Mengen Wasser ab und wird spröde. Beispielsweise verliert die Folie Monosol 8030 in Gegenwart von Natriumbentazon in einem geschlossenen Behältnis einen großen Teil der im Film enthaltenen Restfeuchte. Bei Raumtemperatur geht diese von anfänglich 14 % auf 6 % im Gleichgewichtszustand zurück. Die Folge ist eine Versprödung des Films und ein Platzen des Beutels bei mechanischer Belastung wie Transport, Stoßen und Belasten. Die Ergebnisse von Modellversuchen sind in der folgenden Tabelle zusammegefaßt.

| Art des Salzes | Temperatur | Eigenschaften des Folienbeutels |
|---|---|---|
| Natriumsalz | 20°C | spröde, brüchig |
| | 30°C | spröde, brüchig |
| | | |
| Magnesiumsalz | 20°C | elastisch, stabil |
| | 30°C | elastisch, stabil |

### Anwendungsbeispiele (herbizide Wirksamkeit)

Die herbizide Wirkung der Salze I ließ sich durch Gewächshausversuche zeigen:

Als Kulturgefäße dienten Plastikblumentöpfe mit lehmigem Sand mit etwa 3,0 Gew.-% Humus als Substrat. Die Samen der Testpflanzen wurden nach Arten getrennt eingesät.

Bei Vorauflaufbehandlung wurden die in Wasser verteilen Wirkstoffe direkt nach Einsaat mittels fein verteilen der Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkte ein gleichmäßigeres Keimen der Testpflanzen, sofern dies nicht durch die Wirkstoffe beeinträchtigt wurde.

Zum Zwecke der Nachauflaufbehandlung wurden die Testpflanzen je nach Wuchsform bis zu einer Wuchshöhe von 3 bis 15 cm angezogen und erst dann mit den in Wasser verteilen Wirkstoffen behandelt. Die Testpflanzen wurden entweder bereits in den Versuchsgefäßen gesät und aufgezogen, in denen sie behandelt wurden, oder sie wurden als Keimpflanzen getrennt angezogen und einige Tage vor der Behandlung mit den Wirkstoffaufbereitungen in die Versuchsgefäße verpflanzt.

Die Aufwandmenge für die Nachauflaufbehandlung betrug 0,5 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Code | Lateinischer Name | Deutscher Name |
|---|---|---|
| ABUTH | Abutilon theophrasti | Chinesischer Hanf |
| AMARE | Amaranthus retroflexus | Zurückgekrümmter Fuchsschwanz |
| CHEAL | Chenopodium album | Weißer Gänsefuß |
| GALAP | Galium aparine | Klettenlabkraut |
| IPOSS | Ipomoea ssp. | Prunkwindenarten |
| POLPE | Polygonum persicaria | Flohknöterich |
| SINAL | Sinapis alba | Weißer Senf |
| SOLNI | Solanum nigrum | Schwarzer Nachtschatten |
| STEME | Stellaria media | Vogelsternmiere |
| VERSS | Veronica ssp. | Ehrenpreisarten |

Die biologische Wirkung des Magnesiumsalzes (I) im Vergleich zum bekannten Natriumsalz von 3-Isopropyl-2,1,3-benzothiadiazin-4-on-2,2-dioxid (bei gleicher Formulierung) ist in der folgenden Tabelle zusammengestellt.

| Herbizide Wirkung % | | |
|---|---|---|
| | Natriumsalz | Magnesiumsalz |
| ABUTH | 100 | 100 |
| AMARE | 55 | 20 |
| CHEAL | 100 | 100 |
| GALAP | 85 | 85 |
| IPOSS | 60 | 75 |
| POLPE | 100 | 100 |
| SINAL | 100 | 100 |
| SOLNI | 100 | 100 |
| STEME | 100 | 100 |
| VERSS | 20 | 10 |

## Patentansprüche

1. Festes, nicht-hygroskopisches Magnesiumsalz von 3-Isopropyl-2,1,3-benzothiadiazin-4-on- 2,2-dioxid (I).

2. Feststoff-Formulierung, bestehend zu 20 bis 100 Gew.-% aus dem Magnesiumsalz I, gemäß Anspruch 1.

3. Verfahren zur Herstellung des festen, nicht hygroskopischen Magnesiumsalzes I, gemäß Anspruch 1, dadurch gekennzeichnet, daß man 3-Isopropyl-2,1,3-benzothiadiazin-4-on-2,2-dioxid (II) 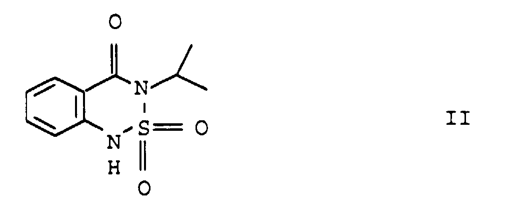 in wäßriger Lösung mit einer anorganischen Magnesiumverbindung behandelt und anschließend den Feststoff in an sich bekannter Weise isoliert und trocknet.

4. Verfahren zur Herstellung einer Feststoff-Formulierung gemäß Anspruch 2, dadurch gekennzeichnet, daß man eine wäßrige Lösung des Magnesiumsalzes I gemäß Anspruch 1 nach dem Verfahren der Sprühtrocknung, Vakuumtrocknung oder nach dem Wirbelbettverfahren trocknet.

5. Mittel zur Bekämpfung von unerwünschtem Pflanzenwachstum, enthaltend eine wirksame Menge des Magnesiumsalzes I gemäß Anspruch 1.

6. Wasserlöslicher oder wasserdispergierbarer Folienbeutel enthaltend ein Magnesiumsalz I gemäß Anspruch 1 oder eine Fest' stoff-Formulierung gemäß Anspruch 2.

7. Verfahren zur Bekämpfung von unerwünschtem Pflanzenwachstum, dadurch gekennzeichnet, daß man die unerwünschten Pflanzen und/oder die von ihnen freizuhaltende Fläche mit einer wirksamen Menge des Magnesiumsalzes I gemäß Anspruch 1 behandelt.

8. Verwendung des Magnesiumsalzes I gemäß Anspruch 1 zur Herstellung von herbiziden Mitteln.

9. Verwendung des Magnesiumsalzes I gemäß Anspruch 1 oder der Feststoff-Formulierung gemäß Anspruch 2 zur Herstellung von wasserlöslichen oder wasserdispergierbaren Folienbeuteln gemäß Anspruch 6.

10. Verwendung des Magnesiumsalzes I gemäß Anspruch 1 zur Herstellung von Wirkstoff-Granulaten.

11. Verwendung des Magnesiumsalzes I gemäß Anspruch 1 zur Bekämpfung von unerwünschtem Pflanzenwuchs.

12. Verwendung der Feststoff-Formulierung gemäß Anspruch 2 zur Bekämpfung von unerwünschtem Pflanzenwuchs.

## Claims

1. A solid, non-hygroscopic magnesium salt of 3-isopropyl-2,1,3-benzothiadiazin-4-one 2,2-dioxide (I).

2. A solid formulation consisting of from 20 to 100% by weight of the magnesium salt I as claimed in claim 1.

3. A process for the preparation of the solid, non-hygroscopic magnesium salt I as claimed in claim 1, wherein 3-isopropyl-2,1,3-benzothiadiazin-4-one 2,2-dioxide (II) 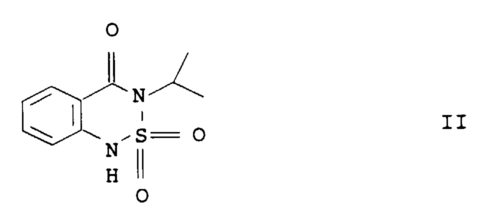 in aqueous solution is treated with an inorganic magnesium compound, and the solid is then isolated and dried in a conventional manner.

4. A process for the preparation of a solid formulation as claimed in claim 2, wherein an aqueous solution of the magnesium salt I as claimed in claim 1 is dried by the spray drying method, by drying under reduced pressure or by the fluidized-bed method.

5. An agent for controlling undesirable plant growth, containing an effective amount of the magnesium salt I as claimed in claim 1.

6. A water-soluble or water-dispersible foil bag containing a magnesium salt I as claimed in claim 1 or a solid formulation as claimed in claim 2.

7. A method for controlling undesirable plant growth, wherein the undesirable plants or the area to be kept free from them is or are treated with an effective amount of the magnesium salt I as claimed in claim 1.

8. Use of the magnesium salt I as claimed in claim 1 for the preparation of herbicides.

9. Use of the magnesium salt I as claimed in claim 1 or of the solid formulation as claimed in claim 2 for the preparation of water-soluble or water-dispersible foil bags as claimed in claim 6.

10. Use of the magnesium salt I as claimed in claim 1 for the preparation of active ingredient granules.

11. Use of the magnesium salt I as claimed in claim 1 for controlling undesirable plant growth.

12. Use of the solid formulation as claimed in claim 2 for controlling undesirable plant growth.

## Revendications

1. Sel de magnésium non hygroscopique solide de 3-iso-propyl-2,1,3-benzothiadiazine-4-on-2,2-dioxyde (I).

2. Formulation solide, constituée par 20 à 100 % en poids de sel de magnésium I, selon la revendication 1.

3. Procédé de préparation du sel de magnésium non hygroscopique solide selon la revendication 1, caractérisé par le fait que l'on traite du 3-iso-propyl-2,1,3-benzothiadiazine-4-on-2,2-dioxyde (II) 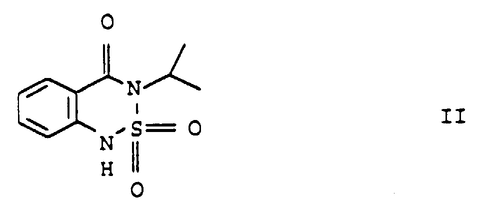 en solution aqueuse, avec un composé de magnésium inorganique et ensuite on isole et sèche le produit solide, de manière connue en soi.

4. Procédé de préparation d'une formulation solide selon la revendication 2, caractérisé par le fait que l'on sèche une solution aqueuse du sel de magnésium I selon la revendication 1, selon le procédé du séchage par pulvérisation, du séchage sous vide ou selon le procédé du lit fluidifié.

5. Produit de lutte contre la croissance des plantes indésirables, contenant une quantité efficace du sel de magnésium I selon la revendication 1.

6. Sac en feuille ou plastique soluble dans l'eau ou dispersable dans l'eau contenant un sel de magnésium I selon la revendication 1 ou une formulation solide selon la revendication 2.

7. Procédé pour lutter contre la croissance des plantes indésirables, caractérisé par le fait que l'on traite les plantes et/ou les surfaces à maintenir exemptes de celles-ci avec une quantité efficace du sel de magnésium I selon la revendication 1.

8. Utilisation du sel de magnésium I selon la revendication 1 pour la préparation d'herbicides.

9. Utilisation du sel de magnésium I selon la revendication 1 ou de la formulation solide selon la revendication 2 pour la préparation de sacs en feuille ou plastiques solubles ou dispersables dans l'eau selon la revendication 6.

10. Utilisation du sel de magnésium I selon la revendication 1 pour la préparation de granulés de matières.

11. Utilisation du sel de magnésium I selon la revendication 1 pour lutter contre la croissance des plantes indésirables.

12. Utilisation de formulation solide selon la revendication 2 pour lutter contre la croissance des plantes indésirables.
